# EUROPEAN PATENT SPECIFICATION

(11) **EP 3 311 736 B1**
(45) Date of publication and mention of the grant of the patent: **26.05.2021**
(21) Application number: 16811755.4
(22) Date of filing: 17.06.2016
(51) Int. Cl.: A61B 5/02, A61B 5/022, A61B 5/021, A61B 5/00, A61B 5/01

(54) **BIOLOGICAL INFORMATION ACQUIRING DEVICE**
VORRICHTUNG ZUR ERFASSUNG BIOLOGISCHER INFORMATIONEN
DISPOSITIF D'ACQUISITION D'INFORMATIONS BIOLOGIQUES

(30) Priority: 19.06.2015 JP 2015124195
(43) Date of publication of application: 25.04.2018
(73) Proprietor: Shinano Kenshi Co., Ltd., Ueda-shi, Nagano 386-0498 (JP); Chuo University, Tokyo 192-0393 (JP)
(72) Inventor: NAKAMURA, Hiroyuki, Ueda-shi Nagano 386-0498 (JP); DOHI, Tetsuji, Tokyo 112-8551 (JP); SATO, Hiroki, Tokyo 112-8551 (JP)
(74) Representative: Schäfer, Matthias W.
(86) International application number: PCT/JP2016/068138
(87) International publication number: WO 2016/204281

(56) References cited:
- JP-A- 2003 235 816
- JP-A- 2006 288 501
- JP-A- 2006 288 501
- JP-A- 2011 239 840
- US-A1- 2009 131 806
- US-A1- 2014 369 530

## Description

### {Technical Field}

The present invention relates to a biological information acquiring device.

### {Background Art}

Blood pressure measurement is available as biological information acquisition of a human body. As blood pressure measurement methods, an oscillometric method and a tonometry method are known.

In the oscillometric method, a cuff is wrapped around the upper arm or wrist to pressure a blood vessel and stop the blood flow temporarily, and then a blood pressure value is measured by checking the pressure in the cuff, which reflects vibration of a blood vessel wall in synchronization with a heart beat, in the process of reducing the pressure in the cuff. On the other hand, in the tonometry method, a blood pressure value is obtained by pressing a sensor with a flat contact pressure against an artery and measuring a fluctuation in the internal pressure of the artery pulsating against the sensor.

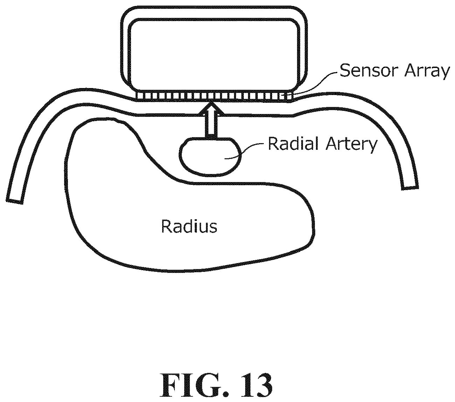

Fig. 13 illustrates a conceptual view of measuring the blood pressure using the tonometry method. The internal pressure of a radial artery is measured by pressing a sensor array formed of many sensors against an area of the wrist corresponding to the radial artery.

### {Citation List}

### {Patent Literature}

{PTL 1}: JP 2011-239840 A
{PTL 2}: JP 2005-253865 A
{PTL 3}: JP 2011-200262 A

Further: US2014/369530 (A1), US2009/131806 (A1), JP2006288501 (A).

### {Summary of Invention}

### {Technical Problem}

Compared to the oscillometric method, the tonometry method has an advantage that pressuring of the blood vessel until the blood flow stops completely is not required. However, a measurement unit used in the tonometry method is formed of the sensor array having tens of channels as illustrated in Fig. 13, where the sensor array needs to be worn at an optimum position above the arterial vessel and apply pressure vertically. For this reason, it is difficult for an ordinary person to use it for health management because wearing of the measurement unit requires assistance of an experienced operator.

Moreover, the tonometry method has a problem of measurement accuracy to be reduced or measurement to become impossible when a body movement causes the wearing condition of the sensor on the human body change to disturb the waveform of a pulse wave acquired. It has thus been required that the body is restrained by using a large-sized body anchor and that a subject is stationary.

Moreover, the measurement unit formed of the sensor array having several tens of channels causes a problem of high power consumption, and the fact that the measurement unit is not portable and the waveform is disturbed by a body movement makes it unsuitable for the measurement unit to measure the blood pressure in exercising.

PTL 1 proposes a blood pressure measurement system employing the tonometry method and using a pressure sensor pressed against a blood vessel wall, but includes no technical concept for obtaining an accurate pulse waveform even when a subject moves his/her body or he/she is in exercising.
PTL 2 and 3 disclose systems for detecting a pulse wave by applying light to a blood vessel, receiving light reflected from the vessel, and performing signal processing. However, it is difficult to perform constant measurement with such systems that are aimed at improving the convenience of measurement and is large in size to restrict a behavior of a subject.

The present invention has been made in view of the above problems, and an object of the present invention to provide a biological information acquiring device that consumes less power, is worn easily, and can acquire an accurate pulse waveform without imposing a burden on a subject.

### {Solution to Problem}

A first aspect of the present invention is a biological information acquiring device for measuring biological information of a subject with a pressure sensor, the device including: one or a plurality of multi-axis pressure sensors for detecting pressure in directions along two or more axes intersecting at a predetermined angle; and an arithmetic unit for calculating outputs of the multi-axis pressure sensors, each of the multi-axis pressure sensors including a signal detection means for detecting a signal of a pressure component for each axis of a pulse wave of the subject, the arithmetic unit including a pulse waveform synthesizing means for synthesizing a pulse waveform based on the signals of the pressure components for respective axes detected by the multi-axis pressure sensors.

The device can include a wearing body to which the multi-axis pressure sensors are mounted and which brings the multi-axis pressure sensors into close contact with the skin of the subject. The multi-axis pressure sensor may be an orthogonal multi-axis pressure sensor that detects pressure components for at least two axes orthogonal to each other. The multi-axis pressure sensor may be an orthogonal triaxial pressure sensor that detects pressure components for three axes.

The pulse waveform synthesizing means may include a blood pressure estimation means for estimating the blood pressure based on the pulse waveform. The device may also include: a display for indicating a more accurate pulse wave measurement position on the basis of the output of the multi-axis pressure sensors; a sensor position moving mechanism provided on the wearing body for moving a position of the multi-axis pressure sensor; and a controller for controlling the sensor position moving mechanism on the basis of the outputs of the multi-axis pressure sensors to move the sensors to the more accurate pulse wave measurement position.

The accurate pulse wave measurement position which is indicated on the display or to which the sensors are moved by the sensor position moving mechanism is preferably a position at which a detected pulse wave converges to a component for one axis out of the pressure components for the axes detected by the multi-axis pressure sensors.

The sensor position moving mechanism preferably includes a mechanism for moving the position of the sensor in the directions along at least an X axis and a Z axis and changing an α angle with respect to a blood vessel subjected to measurement by the multi-axis pressure sensor, where the Z axis corresponds to the direction in which the blood vessel pushes a surface of the skin, the X axis corresponds to the direction perpendicular to an axial direction of the blood vessel and orthogonal to the Z axis, a Y axis corresponds to the axial direction of the blood vessel, the α angle is an inclination of the multi-axis pressure sensor about an X-Z axes plane, and a β angle is the inclination of the multi-axis pressure sensor about a Z-Y axes plane. The sensor position moving mechanism may also move the position of the sensor in the direction along the Y axis and change the β angle.

### {Advantageous Effects of Invention}

The accurate pulse waveform can be acquired even when a body movement is observed. Moreover, the pulse waveform can be acquired constantly with low power consumption.

### {Brief Description of Drawings}

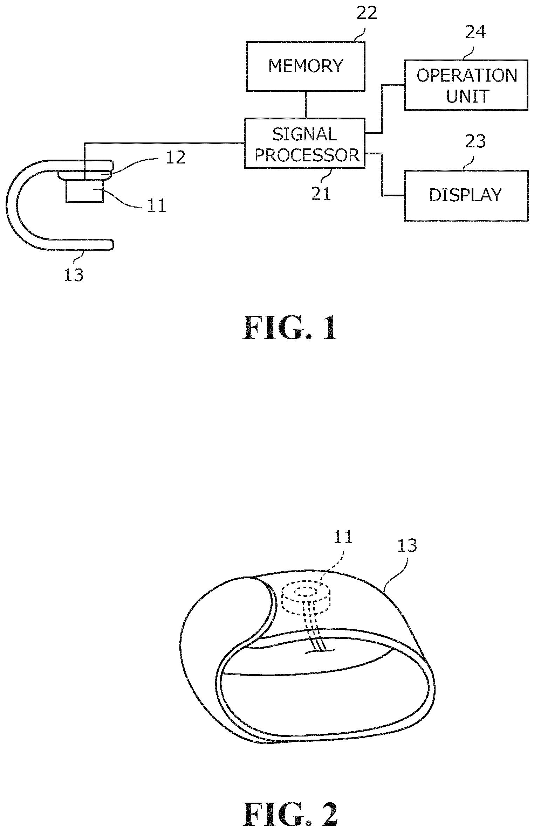

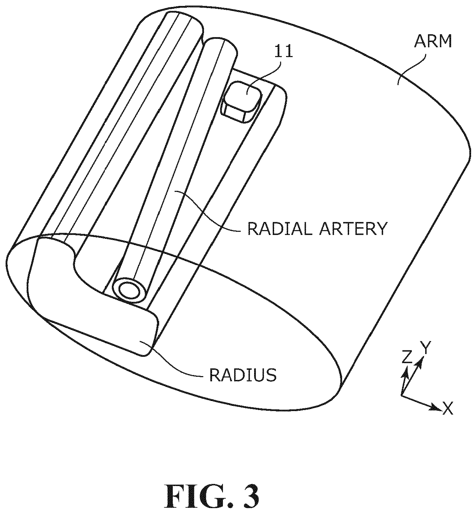

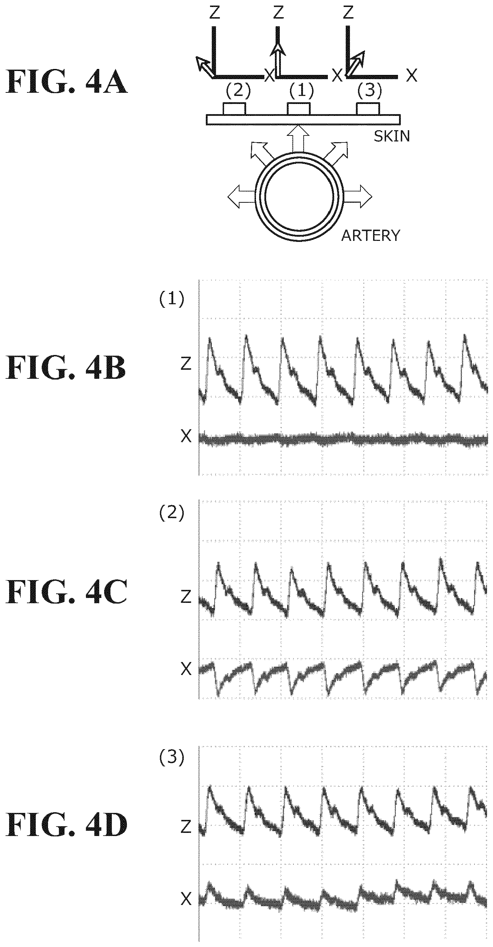

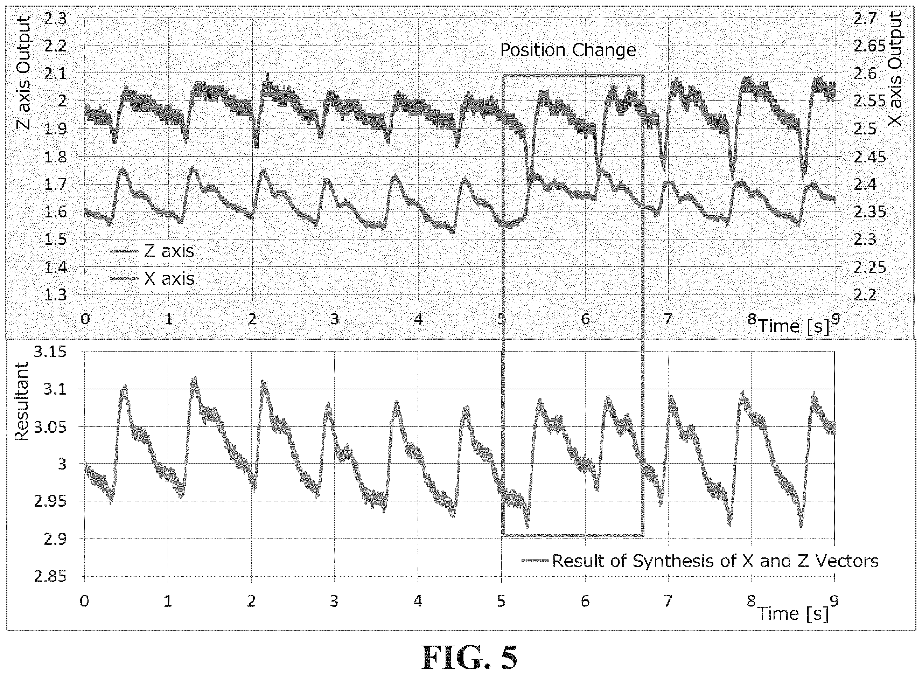

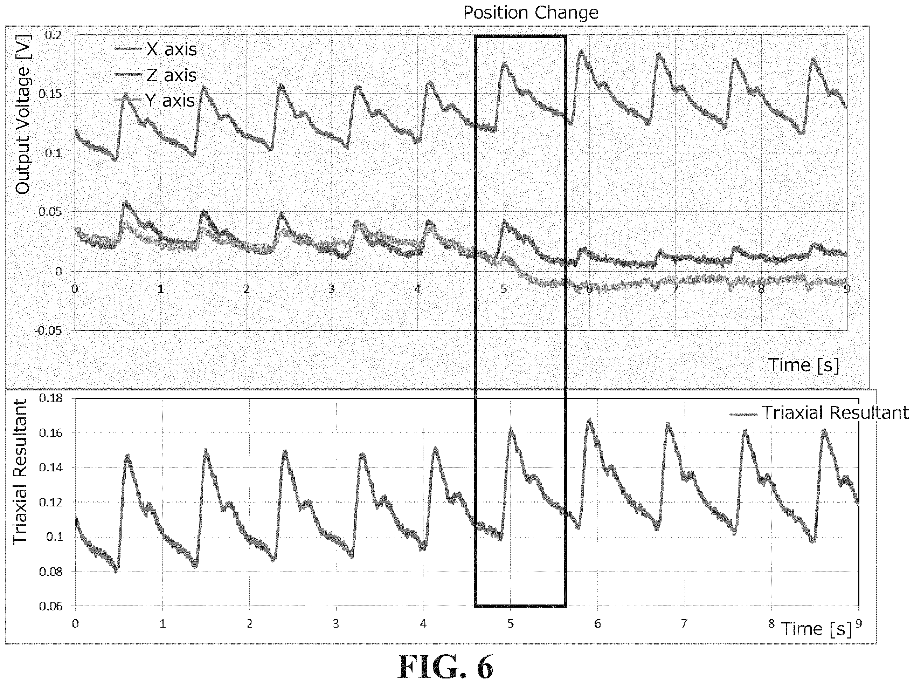

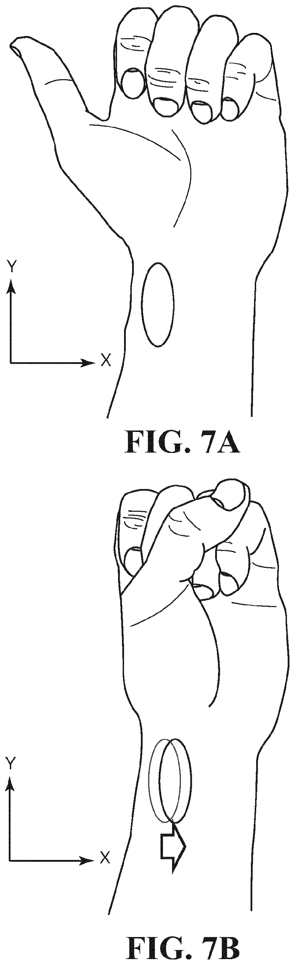

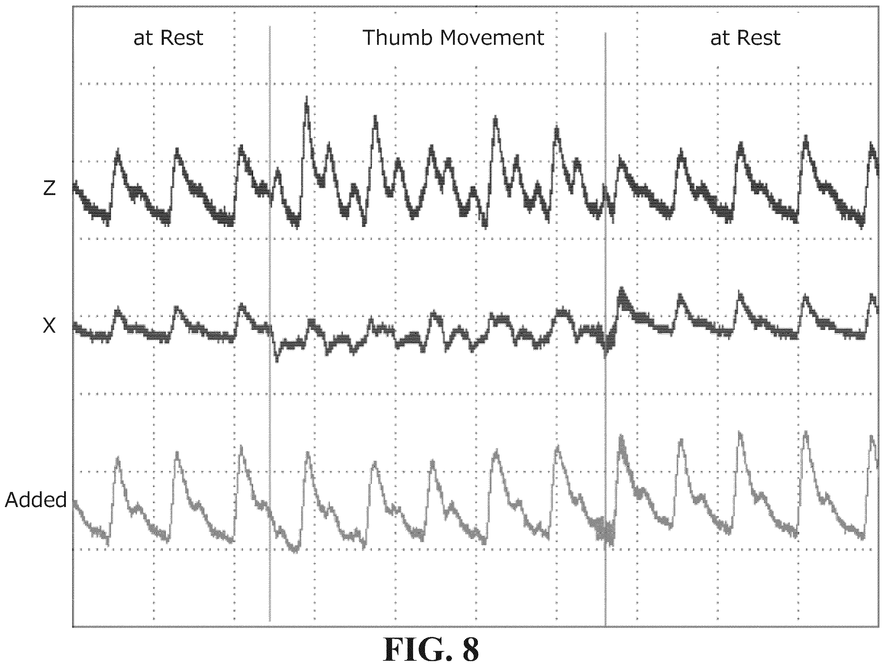

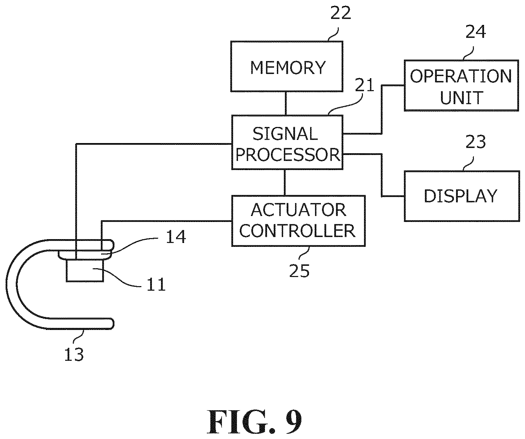

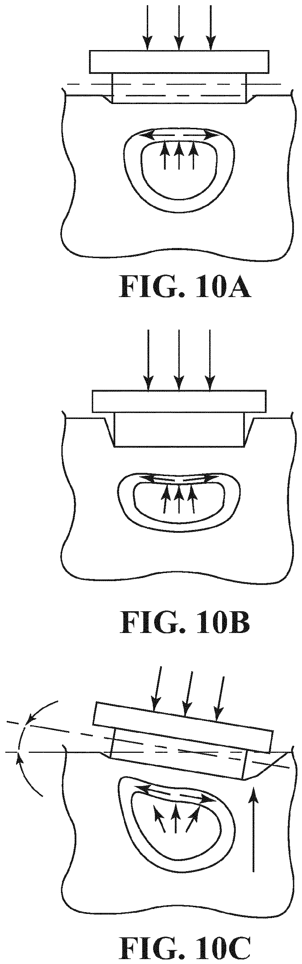

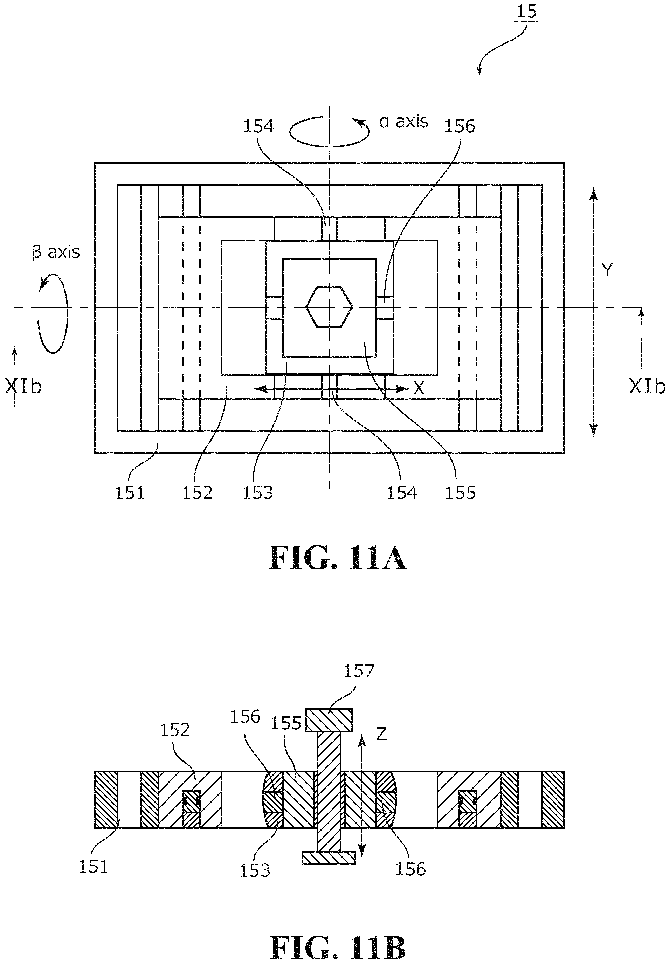

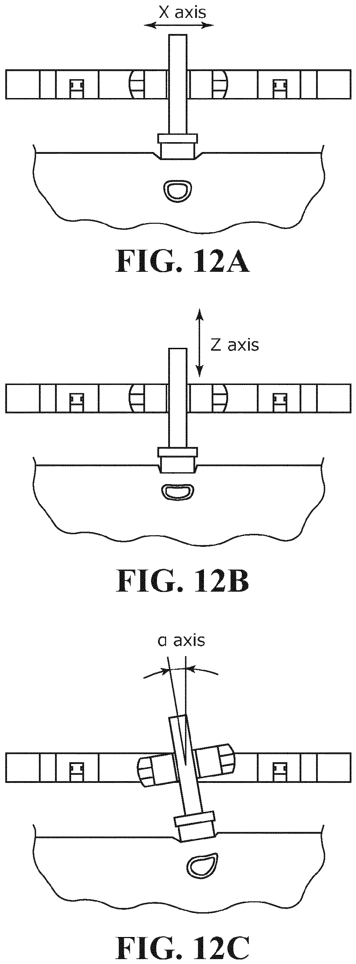

{Fig. 1} Fig. 1 shows a block diagram illustrating a pulse waveform acquisition device according to a first construction.
{Fig. 2} Fig. 2 shows the external appearance of a measurement unit.
{Fig. 3} Fig. 3 shows a view illustrating a principle of measuring a pulse wave of a radial artery.
{Fig. 4} Fig. 4A to Fig. 4D show diagrams illustrating an example of a pressure vector and a detected waveform depending on the position of an artery and a triaxial pressure sensor.
{Fig. 5} Fig. 5 shows a graph illustrating an example of vector synthesis for an X axis and a Z axis when the position of the triaxial pressure sensor is changed.
{Fig. 6} Fig. 6 shows a graph illustrating an example of vector synthesis for the X axis, a Y axis, and the Z axis when the position of the triaxial pressure sensor is changed.
{Fig. 7} Fig. 7A and Fig. 7B show views illustrating a movement of the position of an artery (a body movement) caused by a movement of a thumb.
{Fig. 8} Fig. 8 shows a graph illustrating an example of vector synthesis for the X axis and the Y axis when the body movement is caused by the movement of the thumb.
{Fig. 9} Fig. 9 shows a diagram illustrating the configuration of a pulse waveform acquisition device according to a second construction.
{Fig. 10} Fig. 10A to Fig. 10C show schematic views illustrating a state of force acting depending on the position of a triaxial pressure sensor and a blood vessel.
{Fig. 11} Fig. 11A and Fig. 11B show views illustrating a sensor position moving mechanism of a triaxial pressure sensor according to a third construction.
{Fig. 12} Fig. 12A to Fig. 12C show schematic views illustrating a state of the position of the triaxial pressure sensor adjusted by the sensor position moving mechanism and a blood vessel.
{Fig. 13} Fig. 13 shows a view illustrating a measurement method using a conventional tonometry method.

### {Description of Embodiments}

Exemplary constructions will now be described with reference to the drawings.

Fig. 1 shows a configuration of a pulse wave acquisition device acquiring a pulse wave as biological information according to a first construction, and Fig. 2 shows a view illustrating an example of a configuration of a detection unit to which a sensor of the pulse wave acquisition device is attached for detecting the pulse wave.

As illustrated in Fig. 1, the pulse wave acquisition device according to the first embodiment of the present invention comprises an orthogonal triaxial pressure sensor 11 for detecting pressure in three directions along X, Y, and, Z axes orthogonal to one another as the sensor of the detection unit for detecting the pulse wave of a human body. The orthogonal triaxial pressure sensor (hereinafter referred to as a triaxial pressure sensor) is attached to a wearing body 13 via an elastic body 12. The wearing body 13 is for pressing the triaxial pressure sensor 11 tightly against the skin near the radial artery of the wrist of the human body subjected to measurement. Fig. 2 shows a state in which the triaxial pressure sensor 11 is attached to the wearing body 13. The wearing body 13 is made of resin and is curved to be placed around the wrist of the human body from the side, where the triaxial pressure sensor 11 is attached to a flat inner surface of the wearing body and can be fixed to the wrist. The triaxial pressure sensor 11 is attached to the wearing body 13 via the elastic body 12 such that the triaxial pressure sensor 11 is pressed against the skin with an appropriate pressure.

The wearing body 13 includes a band for fixing the wearing body when one side thereof is opened and fitted on the wrist to press and fix the triaxial pressure sensor 11 against the skin of the wrist. This fixing structure against the wrist is not different in terms of function from a conventional cuff that is fitted to the wrist to acquire a pulse wave. The wearing body 13 may also be made of a soft material such as cloth as with the cuff, so long as the triaxial pressure sensor 11 can be brought into contact with the skin with an appropriate pressure.

Fig. 1 shows a block diagram illustrating the pulse wave acquisition device according to the present embodiment. The pulse wave acquisition device of the present embodiment comprises: a signal processor 21 for receiving detected output of the triaxial pressure sensor 11 attached to the wearing body 13 and calculating a pulse waveform and a blood pressure based on triaxial pressure information being detected; a memory 22 for storing information such as a parameter and a formula used by the signal processor 21 to calculate the blood pressure, waveform data of a calculation result, data from the triaxial pressure sensor 11, and the like; a display 23 for displaying blood pressure information estimated from the pulse waveform or pulse wave being acquired, or displaying operation information such as an operation instruction; and an operation unit 24 for inputting operation information such as start and end of detection.

Here, the display23 and the operation unit 24 may be integrated together. Display and an operational input can be integrated by performing the operational input on a touch panel, for example.

The signal processor 21, the memory 22, the display 23, and the operation unit 24 can be accommodated in a casing separate from the wearing body 13 but may be provided in the wearing body 13 when the device is reduced in size. Although not shown, the casing also has a power supply for operating the pulse wave acquisition device. The power supply may be a battery or may be acquired from a commercial power supply.

The pulse wave acquisition device comprises an external interface (not shown) so that output of the signal processor 21 can be output to an external device via the external interface not shown. The external interface may be used when the pulse wave acquisition device is used as a monitor device of a patient or when the pulse waveform is acquired by an external device such as in acquiring a pulse waveform while a subject is in motion.

Fig. 3 shows a view illustrating a principle of measuring the pulse wave acquired by the pulse wave acquisition device of the present embodiment. The triaxial pressure sensor 11 is placed on the surface of the skin near the radial artery of the wrist and detects pressure in the three directions along the X, Y, and Z axes. The Z axis direction corresponds to an upward direction toward the surface of the skin of the wrist, namely a direction of an arterial pressure exerted toward the skin, the X axis direction corresponds to a lateral direction of the wrist, namely the lateral direction of the wrist with respect to the axis of the radial artery, and the Y axis direction corresponds to a longitudinal direction of the wrist, namely an axial direction of the radial artery.

Fig. 4A to Fig. 4D show views illustrating a relationship between the position of the triaxial pressure sensor 11 with respect to the radial artery (hereinafter also referred to as an artery) and a pulse waveform being detected. Fig. 4A illustrates the position of the triaxial pressure sensor 11 with respect to the radial artery in the lateral direction (X axis direction) of the wrist, and the direction of force detected at that time. Only the Z axis direction and the X axis direction are illustrated in Fig. 4A. When the triaxial pressure sensor 11 is positioned at position (1) in Fig. 4A, the detected force (the pulse wave pressure of the artery) is exerted only in the Z axis direction so that a waveform with a high wave height in the Z axis direction appears large while almost no waveform appears in the X axis direction, as illustrated in a graph of Fig. 4B. On the other hand, at position (2) in Fig. 4A, the detected force is exerted in the direction of an arrow (an upper left direction in the figure), meaning the force is exerted in a positive direction along the Z axis and a negative direction along the X axis. The waveforms in the X and Z axes at this time appear as illustrated in Fig. 4C. The wave height in the Z axis decreases, and the waveform in the X axis is the reverse of the waveform in the Z axis. Moreover, when the triaxial pressure sensor 11 is positioned at position (3) in Fig. 4A, the detected force is exerted in the direction of an arrow (an upper right direction in the figure), so that the waveforms in the Z axis direction and the X axis direction appear as illustrated in Fig. 4D with the waveform appearing in the X axis direction.

As described above, the magnitude of the vectors along the two axes (the X axis and the Z axis) detected by the triaxial pressure sensor 11 varies depending on where the triaxial pressure sensor 11 is positioned with respect to the artery, and the directions of the vectors change as well. The pulse waveform can thus be derived by synthesizing signals, and at the same time the direction of the optimum detection position can be known from the detected output of the triaxial pressure sensor 11.

Note that the origins of the triaxial vectors detected by the triaxial pressure sensor 11 do not strictly coincide with one another and that the vectors do not intersect. This is because, if the triaxial pressure sensor 11 is a sensor using micro electro mechanical systems (MEMS), for example, such a sensor is formed of three sensor elements detecting pressure (stress) in the corresponding axes being the X, Y, and Z axes so that the origins of the vectors of force detected at the corresponding positions of the sensor elements do not coincide with one another. However, the pulse waveforms can be synthesized for measurement with no problem when the shortest distance between the origins of the pressure vectors is roughly equal to the thickness of the artery or the distance between the vectors is less than or equal to the thickness of the artery.

Synthesis of signals when the position of the triaxial pressure sensor 11 is changed will be described.

Fig. 5 shows output waveforms in the Z axis direction and the X axis direction when the triaxial pressure sensor 11 pressed against the radial artery of the wrist is slightly moved to the positive side in the X axis direction, and a waveform obtained as a result of vector synthesis of the two waveforms. Between the two waveforms, the upper waveform represents the waveform in the X axis direction and the lower waveform represents the waveform in the Z axis direction. As shown in Fig. 5, a shift in the position of the triaxial pressure sensor in the X axis direction causes a decrease in the wave height (peak-to-peak) in the Z axis direction and an increase in the wave height in the X axis direction. However, it can be seen from the waveform obtained as a result of vector synthesis of the waveforms in the X axis direction and the Z axis direction that the vector maintains substantially the same waveform as ones before the vector synthesis. This means that, even when the triaxial pressure sensor is placed at a position deviating a little from the radial artery, the pulse wave of the radial artery similar to when the sensor is placed directly above the radial artery can be detected by performing vector synthesis of the waveforms in the Z axis and X axis directions. The pulse waveform can thus be obtained by synthesizing outputs of the two orthogonal axes being the X axis and the Z axis of the triaxial pressure sensor.

Fig. 6 shows output waveforms for the three axes being the X axis, the Y axis, and the Z axis that are synthesized, where the waveforms for the axes correspond to the Z axis, the X axis, and the Y axis from the top at the right end of the graph. A box drawn on the graph indicates a part where the waveform is changed with a change in the position of the triaxial pressure sensor 11. The output waveform for each axis is changed by a change in the position of the triaxial pressure sensor. However, the waveform obtained by synthesizing the waveforms for the three axes has a change in the wave height but no change in the waveform. One can thus see that the pulse waveform can be acquired by synthesizing the output waveforms for the three axes even when the position of the triaxial pressure sensor 11 is changed.

Fig. 7A and Fig. 7B show an example of a body movement of the wrist where the position of the radial artery changes by clasping of the thumb. That is, the position of the radial artery from which pulsation is taken moves in the X axis direction (lateral direction) by clasping of the finger. Fig. 8 shows Z axis output and X axis output of the triaxial pressure sensor 11 at the time of such a body movement as well as the waveform obtained by adding (synthesizing) the Z axis output and the X axis output.

As shown in Fig. 8, the body movement causes little change in the waveform when the Z axis output and the X axis output are synthesized, so that a disturbance in the pulse waveform caused by the body movement can be removed.

In the present configuration, the display can indicate not only the measured blood pressure but also the direction in which the triaxial pressure sensor 11 worn on the wrist is moved to be able to acquire a better pulse wave signal.

The present configuration obtains a vector by synthesizing outputs for the three axes orthogonal to one another and can thus estimate the position of the triaxial pressure sensor 11 where the output for the Z axis is maximized and the outputs for the X axis and the Y axis are minimized. Accordingly, the display 23 may indicate the direction on the X axis and the direction on the Y axis in which the X axis output and the Y axis output of the triaxial pressure sensor 11 are minimized to be able to instruct a movement of the triaxial pressure sensor 11 to a more suitable position. The wearing body may then be moved according to the instruction to be able to place the triaxial pressure sensor at the optimum position for detecting the pulse wave. The output in the Z axis direction is the maximum in this case, meaning that the triaxial pressure sensor 11 is positioned directly above the radial artery, whereby a more preferable pulse waveform can be acquired.

As described above, the embodiment of the present invention can acquire an accurate pulse waveform by synthesizing the detected waveforms for at least two axes even when the position of the sensor is moved or a body movement is observed on the subject. The pulse wave can thus be acquired even while the subject is in motion. Moreover, the device can be worn on the subject to monitor the pulse wave at all times. Furthermore, one triaxial pressure sensor is used to acquire the pulse waveform without using a sensor array including many sensors, whereby a required amount of power is small to be able to reduce the power and size of the pulse wave acquisition device and thus a blood pressure monitor.

Fig. 9 is a view illustrating a second embodiment of the present invention. In the present embodiment, a triaxial pressure sensor 11 is attached to a wearing body 13 via a sensor position moving mechanism 14. Moreover, an actuator controller 25 of the sensor position moving mechanism 14 is connected to a signal processor 21. The sensor position moving mechanism 14 moves the triaxial pressure sensor 11 by using, for example, a piezoelectric element or the like as an actuator (a driving element), and the actuator of the sensor position moving mechanism 14 is driven under the control of the actuator controller 25 and moves the triaxial pressure sensor 11 to a desired position.

In the present embodiment, the direction of pressure of the radial artery can be known from the directions of vector outputs for three axes of the triaxial pressure sensor 11, so that the triaxial pressure sensor 11 is moved by controlling the actuator of the sensor position moving mechanism 14 in X-Y directions in which the vector outputs in the X axis and Y axis directions are minimized and the vector output in the Z axis direction is maximized.

As a result, the triaxial pressure sensor can be automatically moved to the optimum position with a large output in the Z axis by controlling the actuator of the sensor position moving mechanism 14, and can acquire the pulse waveform with a reduced noise component.

The blood pressure can then be estimated by calculation from the detected pulse waveform on the basis of a correlation between the pulse waveform and the blood pressure such as the time from a zero-crossing point of the pulse waveform to a first peak and the time from a first peak to a second peak. This estimation is performed by the signal processor 21, and the blood pressure obtained as a result of the estimation can be output to the display 23 for indication.

With referring Fig. 9, it has been described an example of the sensor position moving mechanism that moves the position of the triaxial pressure sensor 11 in the two directions along X and Y axes. Now, there will be described a third embodiment which adjusts the position of a triaxial pressure sensor in three directions along X, Y, and Z axes and also an inclination of the triaxial pressure sensor with respect to the skin.

Fig. 10A to Fig. 10C schematically shows the pressing force of a triaxial pressure sensor against the skin, the pressure in the blood vessel, and the force caused by a deformation of the blood vessel depending on how the triaxial pressure sensor 11 is positioned with respect to the blood vessel typified by the radial artery. Fig. 10A shows an example in which the triaxial pressure sensor 11 is placed directly above the blood vessel and pressed against the skin with an appropriate force. On this example, tension in the upper part of the blood vessel pressed by the moderate pressing force has only a horizontal component, so that the pressing force of the triaxial pressure sensor 11 and the blood pressure oppose each other in the vertical direction with no component to be a noise appearing in another direction. Fig. 10B shows a state in which the triaxial pressure sensor 11 is pressed against the skin with an excessive pressing force so that the blood vessel is distorted, the blood flow is constricted by the pressure, and the blood pressure cannot be measured accurately. Fig. 10C shows a state in which the triaxial pressure sensor 11 is pressed at an angle against the skin so that the blood vessel is distorted and the blood pressure cannot be measured accurately due to the reaction force of the skin. In Fig. 10C, there is shown the state where the sensor is tilted with respect to the cross section of the blood vessel so that the sensor in this example is tilted in a Z-X axes plane.

Fig. 11A and Fig. 11B show views illustrating a sensor position moving mechanism 15 that can move the triaxial pressure sensor 11 in the directions along the three X, Y, and Z axes and can adjust the inclination of the triaxial pressure sensor about two planes being a ZX plane (which is referred to as an α axis) and a ZY plane (which is referred to as a β axis). The sensor position moving mechanism 15 can adjust the position of the sensor about five axes. Fig. 11A shows a plan view of the sensor position moving mechanism 15, and Fig. 11B shows a cross-sectional view taken along line XIb-XIb of Fig. 11A. The sensor position moving mechanism 15 is provided with an inner frame 152 inside an outer frame 151, where the inner frame can be moved in the Y axis direction by a slider mechanism within the outer frame 151. Moreover, the inner frame 152 is provided with an outer rotation mechanism 153 that can be rotated by a hinge mechanism 154 and moved by a slider mechanism within the inner frame 152. Furthermore, the outer rotation mechanism 153 is provided with an inner rotation mechanism 155 that can be rotated by a hinge mechanism 156. The inner rotation mechanism 155 is provided with a sensor attachment rod 157 such that the triaxial pressure sensor 11 is attached to the tip of the rod.

This configuration allows a movement in the Y axis direction between the outer frame 151 and the inner frame 152 and a movement in the X axis direction between the inner frame 152 and the outer rotation mechanism 153. Moreover, when the ZX plane corresponds to the α axis, the α axis can rotate between the inner frame 152 and the outer rotation mechanism 153, and when the ZY plane corresponds to the β axis, the β axis can rotate between the outer rotation mechanism 153 and the inner rotation mechanism 155. Furthermore, the sensor can be moved in the Z axis direction by a slide mechanism or a thread feeding mechanism of the sensor attachment rod 157.

Fig. 12A to Fig. 12C schematically show how the position of the triaxial pressure sensor 11 is adjusted by the sensor position moving mechanism 15, where Fig. 12A is a view illustrating a positional relationship between the triaxial pressure sensor 11 and a blood vessel when the position of the sensor is adjusted in the X axis direction, Fig. 12B shows a state when the position of the sensor is adjusted in the Z axis direction, and Fig. 12C shows a state when the triaxial pressure sensor is tilted about the α axis. The Y axis direction and the β axis direction are omitted in the drawings. As illustrated in Fig. 12A to Fig. 12C, the use of the sensor position moving mechanism 15 allows for an adjustment such that the triaxial pressure sensor 11 is placed at a position on a human body appropriate for pulse wave measurement, namely directly above the radial artery, and that the sensor applies the optimum pressing force.

Here, when the sensor position moving mechanism is used to perform the position adjustment about the five axes and adjust the inclination of the two axes of the triaxial pressure sensor 11 such that the pulse wave has the maximum amplitude at the time of the position adjustment, there can be obtained the amplitude of the pulse wave about 1.8 times that obtained by an adjustment performed only about the three, X, Y, and Z axes.

Moreover, when the triaxial pressure sensor 11 is an SS22-FFC15 (with the diameter of the pressure receiving surface of the sensor part being 5.5 mm) manufactured by Touchence Inc., an appropriate range of the position adjustment about the five axes is approximately 0 to 1 mm for the X axis, approximately 0 to 3 mm for the Y axis, and, for the Z axis, approximately 4 to 5.5 mm inward from zero being the position at which the triaxial pressure sensor 11 is in contact with the skin. Furthermore, the sensor is positioned appropriately when tiled -15 to -10 degrees about the α axis and -20 to +15 degrees about the β axis. Moreover, the positions in the X axis, the Z axis, and the α axis largely affect a measured value so that a precise adjustment is required for the position adjustment in the directions along the X axis, the Z axis, and the α axis. This can be understood by considering the directions of the blood vessel and the force of the blood pressure acting on the blood vessel detected by the triaxial pressure sensor 11. That is, with the blood pressure acting outward with respect to the cross section of the blood vessel, it is necessary to perform an adjustment such that the force acting in such direction being the force (pressure) in the directions of the X axis, the Z axis, and the α axis which is the ZX plane can be measured appropriately. The pulse wave can certainly be measured more accurately when the sensor is adjusted to an appropriate measurement position about the five axes.

Moreover, the sensor position moving mechanism 15 can be provided on the wearing body 13 to be able to adjust the position of the triaxial pressure sensor 11 about the five axes. Although the sensor position moving mechanism 15 of the third configuration illustrated in Fig. 11A and Fig. 11B has a structure in which the position about the five axes is adjusted manually, an actuator using a piezoelectric element, a motor or the like may be included for each axis to be able to control the position about each axis with the actuator. In this configuration, as in the configuration shown in Fig. 9, an actuator controller 25 can perform control to adjust the position to a position where the detected pulse wave has large amplitude.

Alternatively, the actuators can be included only for the X axis, the Z axis, and the α axis depending on the precision of the position adjustment on the triaxial pressure sensor.

As with the sensor position moving mechanism of the third embodiment, the sensor position moving mechanism shown in Fig. 9 can certainly include a triaxial or five-axis moving mechanism to be moved to the optimum position by the actuators and then move the position of the triaxial pressure sensor 11 to the optimum position. It is also possible to control the movement about only the X axis, the Z axis, and the α axis that greatly affect the measurement.

Here, the significance of the α axis and the β axis in the adjustment of the position of the triaxial pressure sensor will be elaborated.

Some triaxial pressure sensors have high sensitivity and precision in the directions along the X, Y, and Z axes orthogonal to one another depending on the type of the sensor, so that the measurement accuracy can be improved by matching the vector components of the blood pressure with any one of the axial directions.

Moreover, if the vector components of the blood pressure are adjusted to match the Z axis with an input to each of the X and Y axes being lower than or equal to a certain level, the input to the X and Y axes, according to the invention, can be discarded to treat the sensor as a single axis sensor with only the Z axis. In this case, the amount of computation decreases since there is no need to combine forces from the axes, whereby power saving and high-speed processing can be achieved. That is, the sensor can be used as a triaxial pressure sensor when searching for the optimum position, or used as a single axis pressure sensor in performing uninterrupted measurement so that the pulse waveform can be acquired more effectively.

Such sensor is also useful for cases where the composition of a body tissue such as the skin is not uniform. The vector of the blood pressure is usually estimated on the assumption that the composition of the body tissue is uniform, so that an area with the largest vector component of the blood pressure perpendicular to the plane of the skin, namely directly above the blood vessel, is regarded as the optimum position. When the composition of the body tissue is not uniform, however, the area with the largest vector component of the blood pressure perpendicular to the plane of the skin may not always coincide with an area indicating the maximum value of the triaxial resultant. A more accurate pulse waveform can thus be acquired by performing an adjustment about the α axis and the β axis after searching for the area with the largest triaxial resultant and then matching the vector of the blood pressure with the Z axis.

In addition, such sensor is also useful against instability of the position of the blood vessel. For example, as illustrated in Fig. 13, the radius is concave in the cross section of the wrist with the blood vessel being fitted in the concave part. When the triaxial pressure sensor is placed on the surface of the skin against such blood vessel and pressed in the direction perpendicular to the plane of the skin, the blood vessel may escape in the horizontal direction. The position of the sensor is inappropriate when the blood vessel escapes, making it difficult to acquire an accurate pulse waveform. Thus, the pulse waveform can be acquired more stably by preventing the blood vessel from escaping by adjusting the α axis, β axis, X axis, Y axis, and Z axis such that the blood vessel stably fits in the recess of the concave part of the radius while the Z axis of the triaxial pressure sensor matches the blood pressure vector.

Note that the aforementioned embodiment describes an example in which one triaxial pressure sensor is pressed against the radial artery of the wrist to detect the pulse waveform and estimate the blood pressure by calculation.

However, the pulse wave detected by the triaxial pressure sensor may be used for estimating not only the blood pressure but also another health condition as the biological information. For example, flexibility of the blood vessel can be evaluated by using the pulse waveform. Alternatively, a respiratory condition of a subject can be determined to monitor the active state of the sympathetic and parasympathetic nerves. Still alternatively, the level of risk for myocardial infarction can be determined from the pulse waveform. The estimation of these health conditions using the pulse waveform may be performed by modifying the details of the pulse waveform processing.

Furthermore, the position on the human body subjected to measurement need not be a part of the radius but may be any site from which the pulse wave can be acquired. For example, the site may be the temporal region, the cervical region, the knee, the femoral region, or the like.

Moreover, although the aforementioned embodiment describes an example of using one triaxial pressure sensor, the sensor need not be the triaxial pressure sensor since the pulse waveforms can be synthesized and detected when the outputs of at least two axes being the Z axis and the X axis are provided as described above. There may be used two biaxial pressure sensors disposed close to each other, for example.

The pulse waveforms can be synthesized by using the outputs of at least the Z axis and the X axis as described above, so that the pulse waveform can also be acquired by using the biaxial pressure sensors close to each other and synthesizing the outputs thereof to be able to reduce an error caused by a body movement even when a body movement is observed on the subject.

In addition, some triaxial pressure sensors are equipped with a temperature sensor used to compensate for a temperature characteristic. In a configuration, the triaxial pressure sensor is covered with the wearing body and is thermally coupled thereto, so that a thermal equilibrium is attained when the sensor is worn for a certain period of time or longer derived with parameters being the heat (body temperature) generated by the surface of a living body in contact with the wearing body, the heat radiated from the outer surface of the wearing body, and a specific heat of the entire wearing body (and the triaxial pressure sensor).

The temperature sensor of the triaxial pressure sensor can acquire a stable temperature on the surface of the living body when the sensor is worn uninterruptedly over a long period of time as in configurations of the present disclosure. Moreover, a temperature at a deep part of the living body can also be acquired if a correlation between the temperature on the surface of the living body and the temperature at the deep part of the living body is known. In addition, the specific heat of the entire wearing body (and the triaxial pressure sensor) has a function of absorbing the fluctuation in the body temperature along the time axis, so that the temperature acquired by the temperature sensor has a small disturbance and is suitable as secondary information obtained by the long, uninterrupted measurement.

### {Reference Signs List}

- 11: triaxial pressure sensor
- 12: elastic body
- 13: wearing body
- 14, 15: sensor position moving mechanism
- 21: signal processor
- 22: memory
- 23: display
- 24: operation unit
- 25: actuator controller

## Claims

1. A biological information acquiring device for measuring biological information of a subject with a pressure sensor, the device comprising:
one or a plurality of multi-axis pressure sensors (11) for detecting pressure in directions along two or more axes intersecting at a predetermined angle, each of the multi-axis pressure sensors (11) including a signal detection means for detecting a signal of a pressure component for each axis of a pulse wave of the subject;
an arithmetic unit (21) for calculating outputs of the multi-axis pressure sensors (11), the arithmetic unit (21) including a pulse waveform synthesizing means for synthesizing a pulse waveform based on the signals of the pressure components for respective axes detected by the multi-axis pressure sensors (11);
a sensor position moving mechanism (14, 15) for adjusting a position of the multi-axis pressure sensors (11) with respect to the subject, and
a controller (25) for controlling the sensor position moving mechanism (14, 15) on the basis of the outputs of the multi-axis pressure sensors (11) to move the sensors (11) to a more accurate pulse wave measurement position;
the sensor position moving mechanism (14, 15) including actuators for moving the multi-axis pressure sensor (11) in the directions along at least an X axis and a Z axis and for changing an α angle with respect to a blood vessel of the living body subjected to measurement by the multi-axis pressure sensor (11), where the Z axis corresponds to the direction in which the blood vessel pushes a surface of the skin of the subject, the X axis corresponds to the direction perpendicular to an axial direction of the blood vessel and orthogonal to the Z axis, a Y axis corresponds to the axial direction of the blood vessel, the α angle is an inclination of the multi-axis pressure sensor (11) about an X-Z axes plane, and a β angle is the inclination of the multi-axis pressure sensor (11) about a Z-Y axes plane, wherein,
the controller (25) is configured to use triaxial outputs of the multi-axis pressure sensors (11) and the arithmetic unit (21) is configured to use a single axis output from the multi-axis pressure sensors (11).

2. The biological information acquiring device according to claim 1, wherein the sensor position moving mechanism (15) comprises:
an outer frame (151);
an inner frame (152) provided inside the outer frame (151), wherein the inner frame (152) is slidable in one axis direction within the outer frame (151);
an outer rotation mechanism (153) provided in the inner frame (152) rotatable in one angle direction by means of hinge mechanism (154) and slidable in another axis direction within the inner frame (152) by means of a slider mechanism;
an inner rotation mechanism (155) provided within the outer rotation mechanism (153) rotatable in another angle direction by means of hinge mechanism (156); and
wherein the inner rotation mechanism (155) has a sensor attachment rod (157), the multi-axis pressure sensor (11) being attached to a tip of the rod (157).

3. The biological information acquiring device according to claim 1 or 2, further comprising a wearing body (13) to which the multi-axis pressure sensors (11) are mounted and which brings the multi-axis pressure sensors (11) into close contact with the skin of the subject.

4. The biological information acquiring device according to-any one of the preceding claims 1 to 3, wherein
the multi-axis pressure sensor (11) is an orthogonal multi-axis pressure sensor that detects pressure components for at least two axes orthogonal to each other.

5. The biological information acquiring device according to claim 4, wherein the multi-axis pressure sensor (11) is an orthogonal triaxial pressure sensor that detects pressure components for three axes.

6. The biological information acquiring device according to any one of claims 1 to 5, wherein
the pulse waveform synthesizing means of the arithmetic unit (21) includes a blood pressure estimation means for estimating a blood pressure based on the pulse waveform.

7. The biological information acquiring device according to any one of claims 1 to 6, further comprising
a display (23) for indicating a more accurate pulse wave measurement position on the basis of the outputs of the multi-axis pressure sensors (11).

8. The biological information acquiring device according to any one of claims 1 to 7, further comprising:
a controller (25) for controlling the sensor position moving mechanism (14, 15) on the basis of the outputs of the multi-axis pressure sensors (11) to move the sensors (11) to a more accurate pulse wave measurement position.

9. The biological information acquiring device according to any one of claims 7 or 8, wherein the more accurate position is a position at which an output of the multi-axis pressure sensors (11) for the Z-Axis is maximized while outputs of the multi-axis pressure sensors (11) for the other axis are minimized.

10. The biological information acquiring device according to claim 7 or 8, wherein
the accurate pulse wave measurement position is a position at which a detected pulse wave converges to a component for one axis out of the pressure components for the axes detected by the multi-axis pressure sensors (11).

11. The biological information acquiring device according to any one of the preceding claims, wherein the sensor position moving mechanism (14, 15) uses a driving element, in particular a piezoelectric element, for moving the multi-axis pressure sensors (11).

12. The biological information acquiring device according to claim 11, wherein an actuator using a piezoelectric element, or a motor, is included in each axis about which the position of the multi-axis pressure sensors (11) may be adjusted.

13. The biological information acquiring device according to claim 5, wherein the triaxial pressure sensor does not use any sensor array including many sensors.

14. The biological information acquiring device according to any one of the preceding claims 3 to 13, wherein the multi-axis pressure sensors (11) are covered with the wearing body (13) and thermally coupled thereto and wherein the multi-axis pressure sensors (11) are further equipped with a temperature sensor for compensating a temperature characteristic.

## Patentansprüche

1. Vorrichtung zur Erfassung biologischer Information zum Messen biologischer Information einer Testperson mit einem Drucksensor, wobei die Vorrichtung aufweist:
einen oder eine Vielzahl von mehrachsigen Drucksensoren (11) zum Detektieren von Druck in Richtungen entlang von zwei oder mehr Achsen, welche sich unter einem vorgegebenen Winkel schneiden, wobei jeder der mehrachsigen Drucksensoren (11) ein Signaldetektionsmittel zum Detektieren eines Signals einer Druckkomponente für jede Achse einer Pulswelle der Testperson umfasst;
eine Arithmetikeinheit (21) zum Berechnen von Ausgaben der mehrachsigen Drucksensoren (11), wobei die Arithmetikeinheit (21) ein Pulswellenform-Synthetisierungsmittel zum Synthetisieren einer Pulswellenform auf der Grundlage der durch die mehrachsigen Drucksensoren (11) detektierten Signale der Druckkomponenten für die jeweiligen Achsen umfasst;
einen Sensorposition-Bewegungsmechanismus (14, 15) zum Einstellen einer Position der mehrachsigen Drucksensoren (11) in Bezug auf die Testperson und
eine Steuerung (25) zum Steuern des Sensorposition-Bewegungsmechanismus (14, 15) auf der Grundlage der Ausgaben der mehrachsigen Drucksensoren (11), um die Sensoren (11) in eine genauere Pulswellenmessposition zu bewegen;
wobei der Sensorposition-Bewegungsmechanismus (14, 15) Aktoren umfasst, um den mehrachsigen Drucksensor (11) in den Richtungen entlang mindestens einer X-Achse und einer Z-Achse zu bewegen und um einen Winkel α in Bezug auf ein Blutgefäß des lebenden Körpers, welcher der Messung durch den mehrachsigen Drucksensor (11) unterzogen wird, zu ändern, wobei die Z-Achse der Richtung entspricht, in welche das Blutgefäß eine Oberfläche der Haut der Testperson drückt, die X-Achse der Richtung senkrecht zu einer axialen Richtung des Blutgefäßes und orthogonal zu der Z-Achse entspricht, eine Y-Achse der axialen Richtung des Blutgefäßes entspricht, der Winkel α eine Neigung des mehrachsigen Drucksensors (11) um eine X-Z-Achsenebene ist und ein Winkel ß die Neigung des mehrachsigen Drucksensors (11) um eine Z-Y-Achsenebene ist,
wobei
die Steuerung (25) dafür ausgelegt ist, um dreiachsige Ausgaben der mehrachsigen Drucksensoren (11) zu verwenden, und die Arithmetikeinheit (21) dafür ausgelegt ist, um eine einachsige Ausgabe von den mehrachsigen Drucksensoren (11) zu verwenden.

2. Vorrichtung zur Erfassung biologischer Information nach Anspruch 1, wobei der Sensorposition-Bewegungsmechanismus (15) aufweist:
einen äußeren Rahmen (151);
einen inneren Rahmen (152), welcher innerhalb des äußeren Rahmens (151) vorgesehen ist,
wobei der innere Rahmen (152) in einer Achsenrichtung innerhalb des äußeren Rahmens (151) verschiebbar ist;
einen in dem inneren Rahmen (152) vorgesehenen äußeren Drehmechanismus (153), welcher in einer Winkelrichtung mit Hilfe von einem Gelenkmechanismus (154) drehbar und in einer anderen Achsenrichtung innerhalb des inneren Rahmens (152) mit Hilfe von einem Schiebermechanismus verschiebbar ist;
einen innerhalb des äußeren Drehmechanismus (153) vorgesehenen inneren Drehmechanismus (155), welcher in einer anderen Winkelrichtung mit Hilfe von einem Gelenkmechanismus (156) drehbar ist; und
wobei der innere Drehmechanismus (155) eine Sensorbefestigungsstange (157) aufweist, wobei der mehrachsige Drucksensor (11) an einem Kopf der Stange (157) angebracht ist.

3. Vorrichtung zur Erfassung biologischer Information nach Anspruch 1 oder 2, ferner aufweisend einen tragenden Körper (13), an welchem die mehrachsigen Drucksensoren (11) montiert sind und welcher die mehrachsigen Drucksensoren (11) in engen Kontakt mit der Haut der Testperson bringt.

4. Vorrichtung zur Erfassung biologischer Information nach einem der vorhergehenden Ansprüche 1 bis 3, wobei
der mehrachsige Drucksensor (11) ein orthogonaler mehrachsiger Drucksensor ist, welcher Druckkomponenten für mindestens zwei zueinander orthogonale Achsen detektiert.

5. Vorrichtung zur Erfassung biologischer Information nach Anspruch 4, wobei der mehrachsige Drucksensor (11) ein orthogonaler dreiachsiger Drucksensor ist, welcher Druckkomponenten für drei Achsen detektiert.

6. Vorrichtung zur Erfassung biologischer Information nach einem der Ansprüche 1 bis 5, wobei
das Pulswellenform-Synthetisierungsmittel der Arithmetikeinheit (21) ein Blutdruck-Abschätzungsmittel zum Abschätzen eines Blutdrucks auf der Grundlage der Pulswellenform umfasst.

7. Vorrichtung zur Erfassung biologischer Information nach einem der Ansprüche 1 bis 6, ferner aufweisend
eine Anzeige (23) zum Angeben einer genaueren Pulswellenmessposition auf der Grundlage der Ausgaben der mehrachsigen Drucksensoren (11).

8. Vorrichtung zur Erfassung biologischer Information nach einem der Ansprüche 1 bis 7, ferner aufweisend:
eine Steuerung (25) zum Steuern des Sensorposition-Bewegungsmechanismus (14, 15) auf der Grundlage der Ausgaben der mehrachsigen Drucksensoren (11), um die Sensoren (11) in eine genauere Pulswellenmessposition zu bewegen.

9. Vorrichtung zur Erfassung biologischer Information nach einem der Ansprüche 7 oder 8, wobei die genauere Position eine Position ist, an welcher eine Ausgabe der mehrachsigen Drucksensoren (11) für die Z-Achse maximiert ist, während Ausgaben der mehrachsigen Drucksensoren (11) für die andere Achse minimiert sind.

10. Vorrichtung zur Erfassung biologischer Information nach Anspruch 7 oder 8, wobei die genaue Pulswellenmessposition eine Position ist, an welcher eine detektierte Pulswelle zu einer Komponente für eine Achse aus den durch die mehrachsigen Drucksensoren (11) detektierten Druckkomponenten für die Achsen konvergiert.

11. Vorrichtung zur Erfassung biologischer Information nach einem der vorhergehenden Ansprüche, wobei der Sensorposition-Bewegungsmechanismus (14, 15) ein Antriebselement verwendet, insbesondere ein piezoelektrisches Element, um die mehrachsigen Drucksensoren (11) zu bewegen.

12. Vorrichtung zur Erfassung biologischer Information nach Anspruch 11, wobei ein Aktor, welcher ein piezoelektrisches Element oder einen Motor verwendet, in jeder Achse miteingeschlossen ist, um welche die Position der mehrachsigen Drucksensoren (11) eingestellt werden kann.

13. Vorrichtung zur Erfassung biologischer Information nach Anspruch 5, wobei der dreiachsige Drucksensor keine Sensoranordnung verwendet, welche viele Sensoren umfasst.

14. Vorrichtung zur Erfassung biologischer Information nach einem der vorhergehenden Ansprüche 3 bis 13, wobei die mehrachsigen Drucksensoren (11) mit dem tragenden Körper (13) bedeckt und thermisch damit gekoppelt sind und wobei die mehrachsigen Drucksensoren (11) ferner mit einem Temperatursensor ausgerüstet sind, um eine Temperaturkenngröße zu kompensieren.

## Revendications

1. Un dispositif d'acquisition de données biologiques pour mesurer les informations biologiques d'un sujet avec un capteur de pression, le dispositif comprenant :
un ou une multitude de capteurs de pression multiaxes (11) pour détecter la pression dans des directions le long d'un ou deux axes s'entrecoupant à un angle prédéterminé, chacun des capteurs de pression multiaxes (11) comprenant un moyen de détection de signal pour la détection d'un signal d'un composant de pression pour chaque axe d'une onde de pouls du sujet ;
une unité arithmétique (21) pour le calcul des signaux de sortie des capteurs de pression multiaxes (11), l'unité arithmétique (21) comprenant un moyen de synthèse d'une forme d'onde de pouls pour synthétiser une forme d'onde de pouls sur la base des signaux des composants de pression pour des axes respectifs détectés par les capteurs de pression multiaxes (11);
un mécanisme de déplacement de la position de capteur (14, 15) pour ajuster la position des capteurs de pression multiaxes (11) par rapport au sujet ; et
un contrôleur (25) pour contrôler le mécanisme de déplacement de la position de capteur (14, 15) sur la base des signaux de sortie des capteurs de pression multiaxes (11) pour déplacer les capteurs (11) pour une position de mesure d'onde de pouls plus précise ;
le mécanisme de déplacement de la position de capteur (14, 15) comprenant des actionneurs pour déplacer le capteur de pression multiaxes (11) dans les directions le long d'au moins un axe X et un axe Y et pour changer un angle α par rapport à un vaisseau sanguin de l'organisme vivant soumis à la mesure par le capteur de pression multiaxes (11), où l'axe Z correspond à la direction dans laquelle le vaisseau sanguin pousse une surface de la peau du sujet, l'angle α correspond à la direction perpendiculaire à une direction axiale du vaisseau sanguin et orthogonale à l'axe Z, un axe Y correspond à la direction axiale du vaisseau sanguin, l'angle α est une inclinaison du capteur de pression multiaxes (11) sur un plan d'axes X-Z, et un angle β est l'inclinaison du capteur de pression multiaxes (11) sur un plan d'axes Z-Y,
étant précisé que
le contrôleur (25) est configuré de sorte à utiliser les signaux de sortie triaxiale des capteurs de pression multiaxes (11) et l'unité arithmétique (21) est configurée pour utiliser un signal de sortie d'axe simple des capteurs de pression multiaxes (11).

2. Le dispositif d'acquisition de données biologiques selon la revendication 1, étant précisé que le mécanisme de déplacement de la position de capteur (15) comprend :
un cadre externe (151) ;
un cadre interne (152) disposé à l'intérieur du cadre externe (151), étant précisé que le cadre interne (152) peut coulisser dans une direction d'axe dans le cadre externe (151) ;
un mécanisme de rotation externe (153) disposé dans le cadre interne (152) pivotable dans une direction d'angle au moyen d'un mécanisme de charnière (154) et coulissant dans une autre direction d'axe dans le cadre interne (152) au moyen d'un mécanisme coulissant ;
un mécanisme de rotation interne (155) disposé dans le mécanisme de rotation externe (153) pivotable dans une autre direction d'angle au moyen d'un mécanisme de charnière (156) ; et
étant précisé que le mécanisme de rotation interne (155) présente une barre d'attache de capteur (157), le capteur de pression multiaxes (11) étant attaché à la pointe de la barre (157).

3. Le dispositif d'acquisition de données biologiques selon la revendication 1 ou 2, comprenant en outre un corps support (13) sur lequel les capteurs de pression multiaxes (11) sont montés et qui place les capteurs de pression multiaxes (11) en contact étroit avec la peau du sujet.

4. Le dispositif d'acquisition de données biologiques selon l'une quelconque des revendications précédentes de 1 à 3, étant précisé que
le capteur de pression multiaxes (11) est un capteur de pression multiaxes orthogonal qui détecte les composants de pression pour aux moins deux axes orthogonaux l'un par rapport à l'autre.

5. Le dispositif d'acquisition de données biologiques selon la revendication 4, étant précisé que
le capteur de pression multiaxes (11) est un capteur de pression multiaxes orthogonal qui détecte les composants de pression pour trois axes.

6. Le dispositif d'acquisition de données biologiques selon l'une quelconque des revendications 1 à 5, étant précisé que
le moyen de synthèse d'une forme d'onde de pouls de l'unité arithmétique (21) comprend un moyen d'estimation de la pression sanguine pour estimer une pression sanguine sur la base de la forme d'onde de pouls.

7. Le dispositif d'acquisition de données biologiques selon l'une quelconque des revendications 1 à 6, comprenant en outre
un écran (23) pour indiquer une position de mesure de l'onde pouls plus précise sur la base des signaux de sortie des capteurs de pression multiaxes (11).

8. Le dispositif d'acquisition de données biologiques selon l'une quelconque des revendications 1 à 7, comprenant en outre
un contrôleur (25) pour contrôler le mécanisme de déplacement de la position de capteur (14, 15) sur la base des signaux de sortie des capteurs de pression multiaxes (11) pour déplacer les capteurs (11) pour une position de mesure d'onde de pouls plus précise.

9. Le dispositif d'acquisition de données biologiques selon l'une quelconque des revendications 7 ou 8, étant précisé que la position plus précise est une position où un signal de sortie des capteurs de pression multiaxes (11) pour l'axe Z est maximisé tandis que les signaux de sortie des capteurs de pression multiaxes (11) pour les autres axes sont minimisés.

10. Le dispositif d'acquisition de données biologiques selon la revendication 7 ou 8, étant précisé que
la position de mesure d'onde de pouls précise est une position où une onde de pouls converge vers un composant pour un axe des composants de pression pour les axes détectés par les capteurs de pression multiaxes (11).

11. Le dispositif d'acquisition de données biologiques selon l'une quelconque des revendications précédentes, étant précisé que le mécanisme de déplacement de la position de capteur (14, 15) utilise un élément d'entraînement, en particulier un élément piézoélectrique, pour déplacer les capteurs de pression multiaxes (11).

12. Le dispositif d'acquisition de données biologiques selon la revendication 11, étant précisé qu'un actionneur utilisant un élément piézoélectrique, ou un moteur, est inclus dans chaque axe sur lequel la position des capteurs de pression multiaxes (11) peut être ajustée.

13. Le dispositif d'acquisition de données biologiques selon la revendication 5, étant précisé que le capteur de pression triaxial n'utilise pas de groupe de capteurs comprenant de nombreux capteurs.

14. Le dispositif d'acquisition de données biologiques selon l'une quelconque des revendications précédentes 3 à 13, étant précisé que les capteurs de pression multiaxes (11) sont recouverts par le corps support (13) et couplé thermiquement à lui et étant précisé que les capteurs de pression multiaxes (11) sont en outre équipés d'un capteur de température pour compenser une caractéristique de température.
